(19) Europäisches Patentamt
European Patent Office
Office européen des brevets

(11) Veröffentlichungsnummer: **0 213 377**
**B1**

(12) # EUROPÄISCHE PATENTSCHRIFT

(45) Veröffentlichungstag der Patentschrift: **15.02.89**

(51) Int. Cl.⁴: **C 07 F 9/65,** C 07 D 239/36, A 01 N 57/08

(21) Anmeldenummer: **86110263.0**

(22) Anmeldetag: **25.07.86**

(54) **Phosphor (phosphon)-säureester.**

(30) Priorität: **07.08.85 DE 3528264**

(43) Veröffentlichungstag der Anmeldung: **11.03.87 Patentblatt 87/11**

(45) Bekanntmachung des Hinweises auf die Patenterteilung: **15.02.89 Patentblatt 89/7**

(84) Benannte Vertragsstaaten: **AT BE CH DE FR GB IT LI NL**

(56) Entgegenhaltungen:
**FR-A-2 230 651**
**US-A-4 486 422**
**US-A-4 558 039**

(73) Patentinhaber: **BAYER AG, Konzernverwaltung RP Patentabteilung, D-5090 Leverkusen 1 Bayerwerk (DE)**

(72) Erfinder: **Maurer, Fritz, Dr., Röberstrasse 8, D-5600 Wuppertal 1 (DE)**
Erfinder: **Baasner, Bernd, Dr, Hamberger Strasse 27d, D-5090 Leverkusen 3 (DE)**
Erfinder: **Homeyer, Bernhard, Dr., Obere Strasse 28, D-5090 Leverkusen 3 (DE)**
Erfinder: **Behrenz, Wolfgang, Dr., Untergründemich 14, D-5063 Overath (DE)**
Erfinder: **Stendel, Wilhelm, Dr., In den Birken 55, D-5600 Wuppertal (DE)**

EP 0 213 377 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft neue 2-Trifluormethyl-pyrimidin-5-yl-Phosphor(phosphon)säureester, Verfahren zu ihrer Herstellung und ihre Verwendung als Schädlingsbekämpfungsmittel, insbesondere als Insektizide und Nematizide.

Es ist bekannt, daß bestimmte O-Pyrimidinylphosphor-(phosphon)säureester bzw. -esteramide, wie z. B. O,O-Diethyl-O-(2-tert.-butyl-pyrimidin-5-yl)-thionophosphorsäureester, O-Ethyl-O-(2-i-propyl-pyrimidin-5-yl)-N-i-propyl-thionophosphorsäureesteramid und O-Ethyl-O-i-propyl-O-(2-tert.-butyl-pyrimidin-5-yl)-säureester, insektizid wirksam sind (vgl. DE-OS-2 643 262, DE-OS-3 317 824, US-PS-4 486 422 und US-PS-4 429 125).

Die Wirkung dieser bekannten Verbindungen ist jedoch unter bestimmten Umständen, insbesondere bei niedrigen Wirkstoffkonzentrationen und Aufwandmengen nicht immer voll zufriedenstellend.

Es wurden nun neue O-(2-Trifluormethyl-pyrimidin-5-yl)-phosphor(phosphon)-säureester der Formel (I)

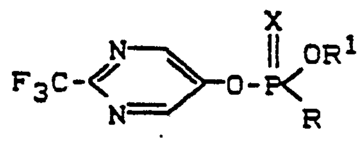

(I)

gefunden, in welcher

X für Sauerstoff oder Schwefel steht,

R für gegebenenfalls substituierte Reste aus der Reihe Alkyl, Alkoxy, Alkylthio, Aryl, Amino, Alkylamino oder Dialkylamino steht und

$R^1$ für gegebenenfalls substituiertes Alkyl oder Aryl steht,

ausgenommen der Fall, in welchem

X für Schwefel steht,

R für Amino steht und

$R^1$ für n-Butyl steht.

Man erhält die neuen O-(2-Trifluormethyl-pyrimidin-5-yl)-phosphor(phosphon)säureester der Formel (I), wenn man 5-Hydroxy-2-trifluormethyl-pyrimidin der Formel (II)

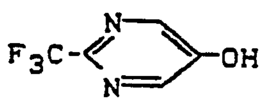

(II)

oder die entsprechenden Alkalimetall-, Erdalkalimetall- oder Ammoniumsalze mit Halogeniden der Formel (III)

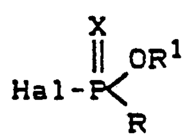

(III)

in welcher

R, $R^1$ und X die oben angegebenen Bedeutungen haben und

Hal für Halogen (vorzugsweise Chlor oder Brom) steht,

gegebenenfalls in Gegenwart eines Säureakzeptors und gegebenenfalls in Gegenwart eines Verdünnungsmittels umsetzt.

Die neuen O-(2-Trifluormethyl-pyrimidin-5-yl)-phosphor-(phosphon)säureester der Formel (I) zeichnen sich durch hohe Wirksamkeit als Schädlingsbekämpfungsmittel, insbesondere durch ihre hervorragende insektizide und nematizide Wirkung aus.

Die gegebenenfalls substituierten Alkylreste R und $R^1$ sowie die Alkylteile der gegebenenfalls substituierten Alkoxy-, Alkylthio-, Alkylamino- und Dialkylaminoreste R können verzweigt oder unverzweigt sein und enthalten vorzugsweise jeweils 1 bis 8, insbesondere 1 bis 6 und besonders bevorzugt 1 bis 4 Kohlenstoffatome. Beispielhaft seien genannt: gegebenenfalls substituiertes Methyl, Ethyl, n-Propyl, iso-Propyl, n-Butyl, iso-Butyl, sec.-Butyl, tert.-Butyl, Methoxy, Ethoxy, n-Propoxy, iso-Propoxy, n-Butoxy, sec.-Butoxy, iso-Butoxy, tert.-Butoxy, Methylthio, Ethylthio, n-Propylthio, iso-Propylthio, n-Butylthio; iso-Butylthio, sec.-Butylthio, tert.-Butylthio, (Di)Methylamino, (Di)Ethylamino, (Di)n-Propylamino, (Di)iso-Propylamino, (Di)n-Butylamino, (Di)iso-Butylamino, (Di)sec.-Butylamino oder (Di)tert.-Butylamino.

Die gegebenenfalls substituierten Arylreste R und $R^1$ enthalten im Arylteil vorzugsweise 6 bis 10 Kohlenstoffatome. Beispielhaft seien genannt: gegebenenfalls substituiertes Naphthyl oder Phenyl, insbesondere gegebenenfalls substituiertes Phenyl.

Die gegebenenfalls substituierten Reste in der Definition von R und $R^1$ können einen oder mehrere, vorzugsweise 1 bis 3, insbesondere 1 oder 2 gleiche oder verschiedene Substituenten tragen. Beispielhaft seien genannt: Halogene, wie Fluor, Chlor und Brom, Nitro, Cyano sowie Alkyl, Alkoxy und Alkylthio mit jeweils vorzugsweise 1 bis 5, insbesondere 1 bis 3 Kohlenstoffatomen.

Bevorzugt sind die Reste R und $R^1$ jedoch unsubstituiert.

X steht bevorzugt für Schwefel.

Die Erfindung betrifft vorzugsweise Verbindungen der Formel (I), in welcher

X für Sauerstoff oder Schwefel (vorzugsweise Schwefel) steht,

R für Alkyl, Alkoxy, Alkylthio, Alkylamino oder Dialkylamino mit jeweils 1 bis 6 (vorzugsweise 1 bis 4) Kohlenstoffatomen (je Alkylteil) für Amino oder Phenyl steht und

$R^1$ für Alkyl mit 1 bis 6 (vorzugsweise 1 bis 4) Kohlenstoffatomen oder Phenyl steht,

ausgenommen der Fall, in welchem

X für Schwefel steht,

R für Amino steht und

$R^1$ für n-Butyl steht.

Besonders bevorzugt sind die Verbindungen der Formel (I), in welcher

X für Sauerstoff oder Schwefel (vorzugsweise Schwefel) steht,

R für Methyl, Ethyl, n-Propyl, i-Propyl, n-Butyl, i-Butyl, Methoxy, Ethoxy, n-Propoxy, i-Propoxy, n-Butoxy, i-Butoxy, Methylthio, Ethylthio, n-Propylthio, i-Propylthio, n-Butylthio, i-Butylthio, sec.-Butylthio, (Di)Methylamino, (Di)Ethylamino, (Di)n-Propylamino, (Di)i-Propylamino, (Di)n-Butylamino, (Di)iso-Butylamino, Methylethylamino, Methyl-n-propylamino, Methyl-i-propylamino, Methyl-n-butylamino, Methyl-i-butylamino, Ethyl-n-propylamino, Ethyl-i-propylamino, Ethyl-n-butylamino, Ethyl-i-butylamino, n-Propyl-i-propylamino, n-Propyl-n-butylamino, n-Propyl-i-butylamino, i-Propyl-n-butylamino, i-Propyl-i-butylamino, n-Butyl-i-butylamino oder Phenyl steht und

$R^1$ für Methyl, Ethyl, n-Propyl, i-Propyl, n-Butyl, i-Butyl, sec.-Butyl, tert.-Butyl und Phenyl steht.

Besonders wertvoll sind Verbindungen der allgemeinen Formel (I), in welcher

X für Schwefel steht,

R für Alkyl, Alkoxy oder Alkylamino mit jeweils 1 bis 4 Kohlenstoffatomen steht und

$R^1$ für Alkyl mit 1 bis 4 Kohlenstoffatomen steht.

Verwendet man beispielsweise für das erfindungsgemäße Verfahren als Ausgangsstoffe 5-Hydroxy-2-trifluormethylpyrimidin und O-Ethyl-O-n-propyl-thionophosphorsäurediesterchlorid, so kann die entsprechende Reaktion durch das folgende Formelschema skizziert werden:

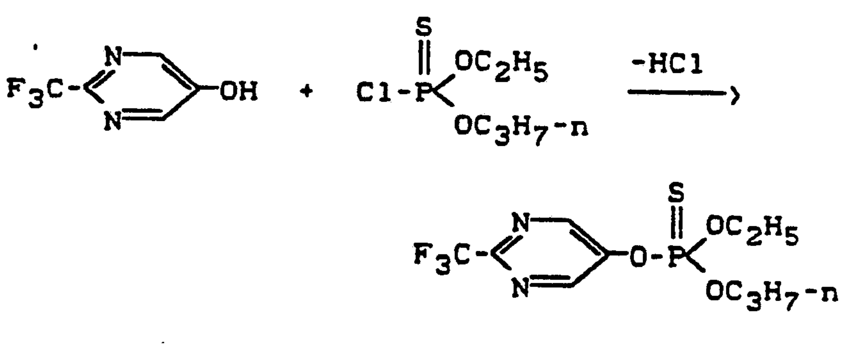


Das für das erfindungsgemäße Verfahren zu verwendende 5-Hydroxy-2-trifluormethyl-pyrimidin bzw. dessen Salze ist durch die Formel (II) definiert. Als Alkali-, Erdalkalimetall- oder Ammoniumsalze werden vorzugsweise Natrium-, Kalium-, Calcium- und Ammoniumsalze verwendet.

Als Beispiel für die Ausgangsstoffe der Formel (II) seien genannt:

das Natrium-, Kalium-, Calcium- und Ammoniumsalz von 5-Hydroxy-2-trifluormethyl-pyrimidin.

Die Verbindung der Formel (II) läßt sich herstellen, indem man 5-Fluor-2-trifluormethyl-pyrimidin der Formel (IV)

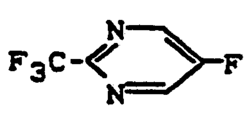

(IV)

in Gegenwart von wäßrigen Säureakzeptoren wie z. B. Natriumhydroxid und in Gegenwart von inerten Verdünnungsmitteln wie z. B. Ethylenglykoldimethylether bei Temperaturen zwischen 50°C und 180°C umsetzt und anschließend durch die Zugabe von Mineralsäuren, wie z. B. Salzsäure ansäuert [vgl. Beispiel (II-1)] und

die Verbindung der Formel (II) nach üblichen Methoden isoliert.

Die Verbindung der Formel (IV) ist bekannt (vgl. z. B. DE-OS-3 402 194).

Die Verbindungen der Formel (III) sind bekannt und/oder können nach allgemein bekannten Verfahren und Methoden hergestellt werden (vgl. z. B. Methoden der organischen Chemie (Houben-Weyl-Müller), 4. Aufl., Band 12/1 (1963), S. 415-420 und S. 560-563; Band 12/2 (1964), S. 274-292, S. 405-408 und S. 607-618, S. 621-622 und S. 755-757; Thieme-Verlag Stuttgart).

Als Beispiele für die Ausgangsstoffe der Formel (III) seien genannt:

Methoxy-methyl-, Methoxy-ethyl-, Methoxy-n-propyl-, Methoxy-i-propyl-, Methoxy-n-butyl-, Methoxy-i-butyl-, Ethoxy-methyl-, Ethoxy-ethyl, Ethoxy-n-propyl-, Ethoxy-i-propyl-, Ethoxy-n-butyl-, Ethoxy-i-butyl-, n-Propoxy-methyl-, n-Propoxy-ethyl-, n-Propoxy-n-propyl-, n-Propoxy-i-propyl-, n-Propoxy-n-butyl-, n-Propoxy-i-butyl-, i-Propoxy-methyl-, i-Propoxy-ethyl-, i-Propoxy-n-propyl-, i-Propoxy-i-propyl-, i-Propoxy-n-butyl-, i-Propoxy-i-butyl-, n-Butoxy-methyl-, n-Butoxy-ethyl-, n-Butoxy-n-propyl-, n-Butoxy-i-propyl-, n-Butoxy-n-butyl-, n-Butoxy-i-butyl-, i-Butoxy-methyl-, i-Butoxy-ethyl-, i-Butoxy-n-propyl-, i-Butoxy-i-propyl-, i-Butoxy-n-butyl-, i-Butoxy-i-butyl-, sec.-Butoxy-methyl, sec.-Butoxy-ethyl, sec.-Butoxy-n-propyl, sec.-Butoxy-i-propyl-, sec.-Butoxy-n-butyl-, sec.-Butoxy-i-butyl-, tert.-Butoxy-methyl-, tert.-Butoxy-ethyl-, tert.-Butoxy-n-propyl-, tert.-Butoxy-i-propyl-, tert.- Butoxy-n-butyl-, tert.-Butoxy-i-butyl-(thiono)phosphonsäureester-chlorid bzw. -bromid; Dimethoxy-, Diethoxy-, Di-n-propoxy-, Di-i-propoxy-, Di-n-butoxy-, Di-i-butoxy-, Methoxy-ethoxy-, Methoxy- n-propoxy-, Methoxy-i-propoxy-, Methoxy-n-butoxy-, Methoxy-i-butoxy-, Methoxy-sec.-butoxy-, Methoxy-tert.-butoxy-, Ethoxy-n-propoxy-, Ethoxy-i-propoxy-, Ethoxy-n-butoxy-, Ethoxy-i-butoxy-, Ethoxy-sec.-butoxy-, Ethoxy-tert.-butoxy-, n-Propoxy-i-propoxy-, n-Propoxy-n-butoxy-, n-Propoxy-i-butoxy-, n-Propoxy-sec.-butoxy-, n-Propoxy-tert.- butoxy-, i-Propoxy-n-butoxy-, i-Propoxy-i-butoxy-, n-Butoxy-i-butoxy-, n-Butoxy-sec.-butoxy-, n-Butoxy- tert.-butoxy-(thiono)phosphorsäureesterchlorid bzw. -bromid;

Methoxy-methylthio-, Methoxy-ethylthio-, Methoxy-n-propylthio-, Methoxy-i-propylthio-, Methoxy-n-butylthio-, Methoxy-i-butylthio-, Ethoxy-methylthio-, Ethoxy-ethylthio-, Ethoxy-n-propylthio-, Ethoxy-i-propylthio-, Ethoxy-n-butylthio-, Ethoxy-i-butylthio-, Ethoxy-sec.-butylthio-, n-Propoxy-methylthio-, n-Propoxy-ethylthio-, n-Propoxy-n-propylthio-, n-Propoxy-i-propylthio-, n-Propoxy-n-butylthio-, n-Propoxy-i-butylthio-, i-Propoxy-methylthio-, i-Propoxy-ethylthio-, i-Propoxy-n-propylthio-, i-Propoxy-i-propylthio-, i-Propoxy-n-butylthio-, i-Propoxy-i-butylthio-, n-Butoxy-methylthio-, n-Butoxy-ethylthio-, n-Butoxy-n-propylthio-, n-Butoxy-i-propylthio-, n-Butoxy-n-butylthio-, n-Butoxy-i-butylthio-, i-Butoxy-methylthio-, i-Butoxy-ethylthio-, i-Butoxy-n-propylthio-, i-Butoxy-i-propylthio-, i-Butoxy-n-butylthio-, i-Butoxy-i-butylthio-(thiono)phosphorsäureester-chlorid bzw. -bromid;

Methoxy-amino-, Ethoxy-amino-, n-Propoxy-amino-, i-Propoxy-amino-, n-Butoxy-amino-, i-Butoxy-amino-, sec.-Butoxy-amino- und tert.-Butoxy-amino-(thiono)phosphorsäureester-chlorid bzw. -bromid;

Methoxy-(di)methylamino-, Methoxy-(di)ethylamino-, Methoxy-(di)-n-propylamino-, Methoxy-(di)i-propylamino-, Methoxy-(di)n-butylamino-, Methoxy-(di)i-butylamino-, Ethoxy-(di)methylamino-, Ethoxy-(di)n-propylamino-, Ethoxy-(di)i-propylamino-, Ethoxy-(di)n-butylamino-, Ethoxy-(di)i-butylamino-, n-Propoxy-(di)methylamino-, n-Propoxy-(di)ethylamino-, n-Propoxy-(di)n-propylamino-, n-Propoxy-(di)i-propylamino-, n-Propoxy-(di)n-butylamino-, n-Propoxy-(di)i-butylamino-, i-Propoxy-(di)methylamino-, i-Propoxy-(di)ethylamino-, i-Propoxy-(di)n-propylamino-, i-Propoxy-(di)i-propylamino-, i-Propoxy-(di)n-butylamino-, i-Propoxy-(di)i-butylamino-, n-Butoxy-(di)methylamino-, n-Butoxy-(di)ethylamino-, n-Butoxy-(di)n-propylamino-, n-Butoxy-(di)i-propylamino-, n-Butoxy-(di)n-butylamino-, n-Butoxy-(di)i-butylamino-, i-Butoxy-(di)methylamino-, i-Butoxy-(di)ethylamino-, i-Butoxy-(di)n-propylamino-, i-Butoxy-(di)i-propylamino-, i-Butoxy-(di)n-butylamino-, i-Butoxy-(di)i-butylamino-, sec.-Butoxy-(di)methylamino-, sec.-Butoxy-(di)ethylamino-, sec.-Butoxy-(di)n-propylamino-, sec.-Butoxy-(di)n-propylamino-, sec.-Butoxy-(di)i-propylamino-, sec.-Butoxy-(di)i-butylamino-, tert.-Butoxy-(di)methylamino-, tert.-Butoxy-(di)ethylamino-, tert.-Butoxy-(di)n-propylamino-, tert.-Butoxy-(di)n-butylamino-(thiono)phosphorsäureester-chlorid bzw. -bromid,

Methoxy-, Ethoxy-, n-Propoxy-, i-Propoxy-, n-Butoxy-, i-Butoxy-, sec.-Butoxy- und tert.-Butoxy-phenyl-(thiono)phosphonsäureester-chlorid bzw. -bromid;

Methyl-, Ethyl-, n-Propyl-, i-Propyl-, n-Butyl-, i-Butyl-, sec.-Butyl- und tert.-Butyl-phenoxy-(thiono)-phosphonsäureesterchlorid bzw. -bromid;

Methoxy-, Ethoxy-, n-Propoxy-, i-Propoxy-, n-Butoxy-, i-Butoxy-, sec.-Butoxy- und tert.-Butoxy-phenoxy-(thiono)phosphorsäureesterchlorid bzw. -bromid,

Methylthio-, Ethylthio-, n-Propylthio-, i-Propylthio-, n-Butylthio-, i-Butylthio-, sec.-Butylthio- und tert.-Butylthio-phenoxy-(thiono)phosphorsäureester-chlorid bzw. -bromid;

Phenyl-, Amino-, (Di)Methylamino-, (Di)Ethylamino-, (Di)n-Propylamino-, (Di)i-Propylamino-, (Di)n-Butylamino- und (Di)i-Butylamino-phenoxy-(thiono)phosphorsäureesterchlorid bzw. -bromid.

Das erfindungsgemäße Verfahren zur Herstellung der neuen Verbindungen der Formel (I) wird bevorzugt unter Verwendung von Verdünnungsmitteln durchgeführt. Als Verdünnungsmittel kommen praktisch alle inerten organischen Lösungsmittel infrage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether und Diglycoldimethylether, Tetrahydrofuran und Dioxan, Ketone, wie Aceton, Methylethyl-, Methylisopropyl- und Methylisobutylketon, Ester, wie Essigsäuremethylester und -ethylester, Nitrile, wie z. B. Acetonitril und Propionitril, Amide, wie z. B. Dimethylformamid, Dimethylacetamid und N-Methylpyrrolidon, sowie Dimethylsulfoxid, Tetramethylensulfon

und Hexamethylphosphorsäuretriamid.

Das Verfahren kann gegebenenfalls in Gegenwart von Säureakzeptoren durchgeführt werden. Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin und Pyridin.

Das erfindungsgemäße Verfahren wird im allgemeinen bei Temperaturen zwischen 0°C und 120°C durchgeführt. Bevorzugt wird der Bereich zwischen 10°C und 100°C. Die Umsetzungen werden im allgemeinen bei Normaldruck durchgeführt.

Zur Durchführung des erfindungsgemäßen Verfahrens werden die Ausgangsstoffe (II) und (III) gewöhnlich annähernd in äquimolaren Mengen eingesetzt. Ein Überschuß der einen oder anderen Reaktionskomponente bringt keine wesentlichen Vorteile.

Die Umsetzungen werden im allgemeinen in einem geeigneten Verdünnungsmittel und gegebenenfalls in Gegenwart eines Säureakzeptors durchgeführt. Die Aufarbeitung geschieht nach üblichen Methoden. Die neuen Verbindungen fallen zum Teil in Form von Ölen an, die sich zum Teil nicht unzersetzt destillieren lassen, jedoch durch sogenanntes 'Andestillieren', d. h. längeres Erhitzen unter vermindertem Druck auf mäßig erhöhte Temperatur von den letzten flüchtigen Anteilen befreit und auf diese Weise gereinigt werden. Zu ihrer Charakterisierung dient der Brechungsindex.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophagus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp..

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrsus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp..

Aus der Ordnung der Arachnida z. B. Scorpio maurus, Latrodectus mactans.

Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe der Formel (I) zeichnen sich durch eine hervorragende insektizide und nematizide Wirksamkeit aus. Sie zeigen insbesondere beim Einsatz als Bodeninsektizide eine hevorragende Wirkung gegen Maden wie z. B. Phorbia antiqua-Maden, gegen Käferlarven, wie z. B. Phaedon cochleariae und Blattläuse, wie z. B. Myzus persicae. Sie zeigen außerdem eine sehr gute Wirksamkeit beim Einsatz gegen Nematoden, wie z. B. Meloidogyne incognita.

Die neuen Verbindungen sind also besonders gut für einen Einsatz zur Bekämpfung von Bodeninsekten und Nematoden geeignet.

Die erfindungsgemäßen Verbindungen können weiterhin bei der Bekämpfung von Hygiene- und Vorratsschädlingen eingesetzt werden.

Weiterhin zeigen die neuen Verbindungen eine blattinsektizide und akarizide Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatisch, Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage; z. B. natürliche Gesteinsmehle, wie Kaoline, Tonerden, Talkum, Kreide, Ouarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z. B. höhere Milchleistungen, höheres Gewicht, schöneres Tierfell, längere Lebensdauer usw. erreicht werden können.

Die Anwendung der erfindungsgemäßen Wirkstoffe geschieht auf diesem Gebiet in bekannter Weise, wie durch äußerliche Anwendung in Form beispielsweise des Tauchens (Dippen), Sprühens (Sprayen), Aufgießen (pour-on and spot-on) und des Einpuderns.

Die biologische Wirksamkeit der erfindungsgemäßen Verbindungen soll anhand der folgenden Beispiele erläutert werden.

**Verwendungsbeispiele**

In den nachfolgenden Verwendungsbeispielen werden die nachstehend angegebenen Verbindungen als Vergleichsverbindungen eingesetzt:

(A) 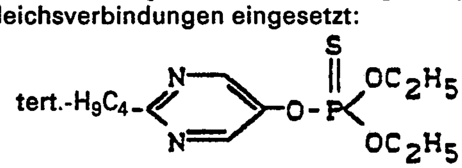

(aus US-PS-4 429 125)

(B) 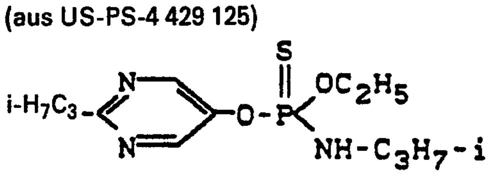

(aus DE-OS-2 643 262)

(C) 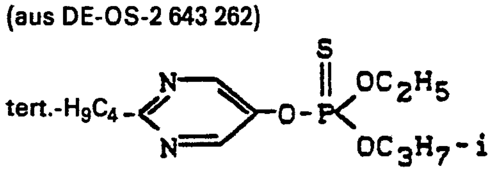

(aus DE-OS-3 317 824)

**Beispiel A**

Grenzkonzentrations-Test / Bodeninsekten

| | |
|---|---|
| Testinsekt: | Phorbia antiqua-Maden im Boden |
| Lösungsmittel: | 3 Gewichtsteile Aceton |
| Emulgator: | 1 Gewichtsteil Alkylarylpolyglykolether |

Zur Herstellung einer zweckmäßigen Wirkstoffzubereitung wermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z. B. die Verbindungen der Beispiele (1) und (2) bei einer beispielhaften Konzentration von 1,25 ppm eine Abtötung von 100 %, während die Vergleichsverbindung (B) bei der gleichen Konzentration 0 % Abtötung ergab.

**Beispiel B**

Grenzkonzentrations-Test / Wurzelsystemische Wirkung

Testinsekt: Phaedon cochleariae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigte z. B. die Verbindung von Beispiel (2) bei einer beispielhaften Konzentration von 10 ppm eine Abtötung von 100 %, während die Vergleichsverbindungen (A) und (B) bei der gleichen Konzentration 0 % Abtötung ergaben.

**Beispiel C**

Grenzkonzentrations-Test / Wurzelsystemische Wirkung

Testinsekt: Myzus persicae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigten z. B. die verbindungen der Beispiele (1) und (2) bei einer beispielhaften Konzentration von 10 ppm eine Abtötung von 100 %, während die Vergleichs-Verbindungen (A) und (B) bei der gleichen Konzentration 0 % Tötung ergaben.

**Beispiel D**

Grenzkonzentrations-Test / Nematoden

Testnematode: Meloidogyne incognita
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27° C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigten z. B. die Verbindungen der Beispiele (1) und (2) bei einer beispielhaften Konzentration von 10 ppm eine Abtötung von 100 %, während die Vergleichsverbindung (c) bei der gleichen Konzentration 0 % Abtötung ergaben.

**Beispiel E**

$LT_{100}$-Test für Dipteren

Testtiere: Musca domestica (resistent)
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %-igen knock down-Effekt notwendig ist.

Bei diesem Test zeigten z. B. die Verbindungen der Herstellungsbeispiele (1), (2) und (3) eine 100 %-ige Wirkung bei einer beispielhaften Wirkstoffkonzentration von 0,02 % nach 40, 90 bzw. 45 Minuten, während die Vergleichsverbindungen (A) und (B) eine 90- bzw. 100 %-ige Wirkung bei der gleichen Konzentration erst nach 6 stunden zeigten.

**Beispiel F**

Test mit Lucilia cuprina resistent-Larven

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^2$ Pferdefleisch und 0,5 ml der Wirkstoffzubereitung enthält. Nach 24 Stunden wird der Abtötungsgrad bestimmt.

Bei diesem Test zeigten z. B. die Verbindungen der Herstellungsbeispiele (1), (2) und (3) bei einer Wirstoffkonzentration von 100ppm eine 100 %-ige Abtötung.

**Herstellungsbeispiele**

**Beispiel 1:**

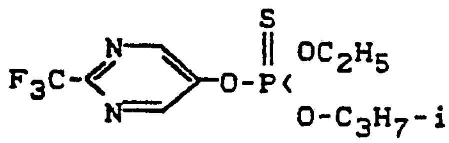

Eine Mischung aus 5 g (0,03 Mol) 5-Hydroxy-2-trifluormethylpyrimidin, 6,9 g (0,05 Mol) Kaliumcarbonat, 50 ml Acetonitril und 6,1 g (0,03 Mol) Thiophosphorsäure-O-ethyl-O-iso-propyl-diesterchlorid wird 2 Stunden bei 20°C gerührt. Nach Zugabe von 150 ml Toluol wird 3-mal mit je 100 ml Wasser ausgeschüttelt. Dann trocknet man die organische Phase über Natriumsulfat und destilliert das Lösungsmittel im Vakuum ab. Der Rückstand wird im Vakuum bei 80°C andestilliert.

Man erhält so 8,8 g (89 % der Theorie) O-Ethyl-O-i-propyl-O-(2-trifluormethyl-pyrimidin-5-yl)-thionophosphorsäureester als gelbes Öl mit einem Brechungsindex $n_D^{22} = 1,4598$.

Analog Beispiel 1 können die folgenden Verbindungen der Formel (I) hergestellt werden:

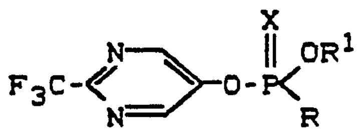

(I)

| Bsp.Nr. | R | $R^1$ | X | Brechungsindex |
|---|---|---|---|---|
| 2 | $i-C_3H_7NH-$ | $C_2H_5-$ | S | $n_D^{22} = 1,4787$ |
| 3 | $CH_3-$ | $CH_3-$ | S | $n_D^{22} = 1,4870$ |
| 4 | $n-C_3H_7-S-$ | $C_2H_5-$ | S | |
| 5 | $C_2H_5O-$ | $C_2H_5-$ | S | |
| 6 | $C_2H_5O-$ | $i-C_3H_7-$ | O | |
| 7 | $n-C_3H_7-S-$ | $C_2H_5-$ | O | |
| 8 | $NH_2-$ | $C_2H_5-$ | S | |
| 9 | $(CH_3)_2N-$ | $C_2H_5-$ | S | |
| 10 | $C_6H_5-$ | $C_2H_5-$ | S | |
| 11 | $C_2H_5O-$ | $C_6H_5-$ | S | |
| 12 | $CH_3O-$ | $CH_3-$ | S | |
| 13 | $i-C_3H_7NH-$ | C2H5- | O | |
| 14 | $sec-C_4H_9O-$ | $C_2H_5-$ | S | |
| 15 | $n-C_3H_7O-$ | $C_2H_5-$ | S | |
| 16 | $C_2H_5O-$ | $i-C_4H_9-$ | S | |
| 17 | $i-C_3H_7O-$ | $CH_3-$ | S | |
| 18 | $C_2H_5-$ | $C_2H_5-$ | S | |

**Ausgangsprodukt der Formel (II)**

**Beispiel (II-1):**

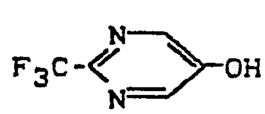

Zu einer Mischung aus 100 ml Ethylenglykoldimethylether, 40 ml Wasser und 25 g 45 %-ige Natronlauge gibt man 20,5 g (0,123 Mol) 5-Fluor-2-trifluormethyl-pyrimidin und erhitzt 2 stunden unter Rückfluß. Dann destilliert man das Lösungsmittel im Vakuum ab, versetzt den Rückstand mit 100 ml Wasser und filtriert vom Ungelösten ab. Das Filtrat wird unter Kühlen bei 5 - 10°C durch Zugabe von konzentrierter Salzsäure auf pH 3,5-4 gebracht. Das ausgefallene Produkt wird abgesaugt und mit wenig kaltem Wasser nachgewaschen.

Man erhält so 15,9 g (79 % der Theorie) 5-Hydroxy-2-trifluormethyl-pyrimidin in Form farbloser Kristalle vom Schmelzpunkt 179-181°C.

**Patentansprüche**

1. O-(2-Trifluormethyl-pyrimidin-5-yl)-phosphor-(phosphon)-säureester der Formel (I)

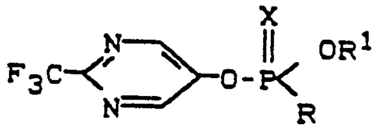

(I)

in welcher
X für Sauerstoff oder Schwefel steht,
R für gegebenenfalls substituierte Reste aus der Reihe Alkyl, Alkoxy, Alkylthio, Aryl, Amino, Alkylamino oder Dialkylamino steht und
$R^1$ für gegebenenfalls substituiertes Alkyl oder Aryl steht,

ausgenommen der Fall, in welchem
X für Schwefel steht,
R für Amino steht und
$R^1$ für n-Butyl steht,

2. Verbindungen der Formel (I) gemäß Anspruch 1,
in welcher
X für Sauerstoff oder Schwefel steht,
R für Alkyl, Alkoxy, Alkylthio, Alkylamino oder Dialkylamino mit jeweils 1 bis 6 Kohlenstoffatomen (je Alkylteil), für Amino oder Phenyl steht und
$R^1$ für Alkyl mit 1 bis 6 Kohlenstoffatomen oder Phenyl steht,

ausgenommen der Fall, in welchem
X für Schwefel steht,
R für Amino steht und
$R^1$ für n-Butyl steht.

3. Verbindungen gemäß den Ansprüchen 1 oder 2, in welchen X in der Formel (I) für Schwefel steht.

4. Verbindungen der Formel (I) gemäß Anspruch 1,
in welcher
X für Schwefel steht,
R für Methyl, i-Propoxy oder i-Propylamino steht und
$R^1$ für Methyl oder Ethyl steht.

5. Verfahren zur Herstellung von O-(2-Trifluormethyl-pyrimidin-5-yl)-phosphor(phoshon)-säureestern der Formel (I)

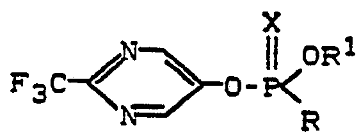

(I)

in welcher
X für Sauerstoff oder Schwefel steht,
R für gegebenenfalls substituierte Reste aus der Reihe Alkyl, Alkoxy, Alkylthio, Aryl, Amino, Alkylamino oder Dialkylamino steht und
$R^1$ für gegebenenfalls substituiertes Alkyl oder Aryl steht,

ausgenommen der Fall, in welchem
X für Schwefel steht,
R für Amino steht und
$R^1$ für n-Butyl steht,
dadurch gekennzeichnet, daß man 5-Hydroxy-2-trifluormethyl-pyrimidin der Formel (II)

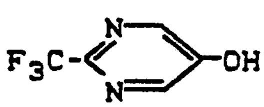

(II)

oder die entsprechenden Alkalimetall-, Erdalkalimetall- oder Ammoniumsalze mit Halogeniden der Formel (III)

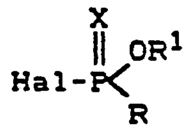

(III)

in welcher
R, $R^1$ und X die oben angegebenen Bedeutungen haben und
Hal für Halogen (vorzugsweise Chlor oder Brom) steht,
gegebenenfalls in Gegenwart eines Säureakzeptors und gegebenenfalls in Gegenwart eines Verdünnungsmittels umsetzt.

6. Schädlingsbekämpfungsmittel, gekennzeichnet durch einen Gehalt an mindestens einer Verbindung der Formel (I) gemäß Anspruch 1 oder 5.

7. Verwendung von Verbindungen der Formel (I) gemäß Anspruch 1 oder 5 zur Bekämpfung von Schädlingen, insbesondere von Insekten und Nematoden.

8. Verfahren zur Bekämpfung von Schädlingen, dadurch gekennzeichnet, daß man Verbindungen der Formel (I) gemäß Anspruch 1 oder 5 auf die Schädlinge, vorzugsweise Insekten und Nematoden oder ihren Lebensraum einwirken läßt.

9. Verfahren zur Herstellung von Schädlingsbekämpfungsmitteln, dadurch gekennzeichnet, daß man Verbindungen der Formel (I) gemäß Anspruch 1 oder 5 mit Streckmitteln und/oder oberflächenaktiven Mitteln vermischt.

## Claims

1. O-(2-Trifluoromethyl-pyrimidin-5-yl)-phosphoric (phosphonic) acid esters of the formula

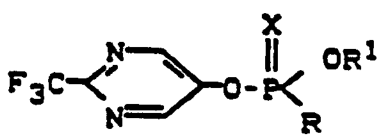

(I)

in which
X represents oxygen or sulphur,
R represents optionally substituted radicals from the series comprising alkyl, alkoxy, alkylthio, aryl, amino, alkylamino and dialkylamino and
$R^1$ represents optionally substituted alkyl or aryl

with the exception of the case in which
X represents sulphur,
R represents amino and
$R^1$ represents n-butyl.

2. Compounds of the formula (I) according to Claim 1,
in which
X represents oxygen or sulphur,
R represents alkyl, alkoxy, alkylthio, alkylamino or dialkylamino with in each case 1 to 6 carbon atoms (per alkyl part), or represents amino or phenyl and
$R^1$ represents alkyl with 1 to 6 carbon atoms or phenyl

with the exception of the case in which
X represents sulphur,
R represents amino and
$R^1$ represents n-butyl.

3. Compounds according to Claims 1 or 2, in which, in formula (I),
X represents sulphur.

4. Compounds of the formula (I) according to Claim 1,
in which

X represents sulphur,
R represents methyl, i-propoxy or i-propylamino and
$R^1$ represents methyl or ethyl.

5. Process for the preparation of O-(2-trifluoromethyl-pyrimidin-5-yl)-phosphoric(phosphonic) acid esters of the formula (I)

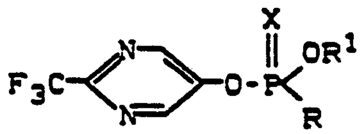

(I)

in which
X represents oxygen or sulphur,
R represents optionally substituted radicals from the series comprising alkyl, alkoxy, alkylthio, aryl, amino, alkylamino and dialkylamino and
$R^1$ represents optionally substituted alkyl or aryl,

with the exception of the case in which
X represents sulphur,
R represents amino and
$R^1$ represents n-butyl

characterized in that 5-hydroxy-2-trifluoromethylpyrimidine of the formula (II)

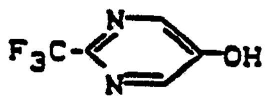

(II)

or the corresponding alkali metal salts, alkaline earth metal salts or ammonium salts, is/are reacted with halides of the formula (III)

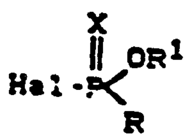

(III)

in which
R, $R^1$ and X have the abovementioned meanings and
Hal represents halogen (preferably chlorine or bromine),
if appropriate in the presence of an acid acceptor and if appropriate in the presence of a diluent.

6. Agents for combating pests, characterized in that they contain at least one compound of the formula (I) according to Claim 1 or 5.

7. Use of compounds of the formula (I) according to Claim 1 or 5 for combating pests, in particular insects and nematodes.

8. Method of combating pests, characterized in that compounds of the formula (I) according to Claim 1 or 5 are allowed to act on the pests, preferably insects and nematodes, or their environment.

9. Process for the preparation of agents for combating pests, characterized in that compounds of the formula (I) according to Claim 1 or 5 are mixed with extenders and/or surface-active agents.

**Revendications**

1. Esters d'acide O-(2-trifluorométhyl-pyrimidin-5-yl)-phosphoro-(phosphonique)de formule (I)

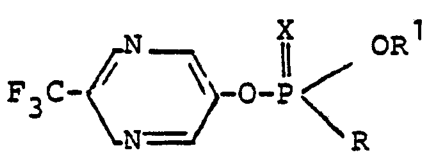

(I)

dans laquelle
X représente un atome d'oxygène ou un atome de soufre

R représente des radicaux éventuellement substitués choisis parmi la série comprenant les radicaux alkyle, alcoxy, alkylthio, aryle, amino, alkylamino ou dialkylamino, et
$R^1$ représente un radical aryle ou un radical alkyle éventuellement substitué,

à l'exception du cas dans lequel
X représente un atome de soufre,
R représente un groupe amino, et
$R^1$ représente un groupe n-butyle.

2. Composés de formule (I) selon la revendication 1, dans laquelle
X représente un atome d'oxygène ou un atome de soufre,
R repésente un radicale alkyle, un radical alcoxy, un radical alkylthio, un radical alkylamino ou un radical dialkylamino contenant chacun 1 à 6 atomes de carbone (par chaque fraction alkyle), un radical amino ou un radical phényle et,
$R^1$ représente un radical alkyle contenant 1 à 6 atomes de carbone, ou un radical phényle,

à l'exception du cas dans lequel
X représente un atome de soufre,
R représente un groupe amino et
$R^1$ représente un groupe n-butyle.

3. Composés selon les revendications 1 ou 2, dans lesquels X de la formule (I) représente un atome de soufre.

4. Composés de formule (I) selon la revendication 1, dans laquelle
X représente un atome de soufre,
R représente un radical méthyle, un radical i-propoxy ou un radical i-propylamino et
$R^1$ représente un radical méthyle ou un radical éthyle.

5. Procédé de préparation d'esters d'acides O-(2-trifluorométhylpyrimidin-5-yl)-phosphoro(phosphoniques) de formule (I)

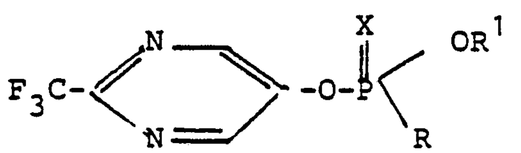

(I)

dans laquelle
X représente un atome d'oxygène ou un atome de soufre,
R représente des radicaux éventuellement substitués choisis parmi la série comprenant des radicaux alkyle, alcoxy, alkylthio, aryle, amino, alkylamino ou dialkylamino, et
$R^1$ représente un radical aryle ou un radical alkyle éventuellement substitué,

à l'exception du cas dans lequel
X représente un atome de soufre,
R représente un groupe amino et
$R^1$ représente un groupe n-butyle,

caractérisé en ce qu'on fait réagir une 5-hydroxy-2-trifluorométhyl-pyrimidine de formule (II)

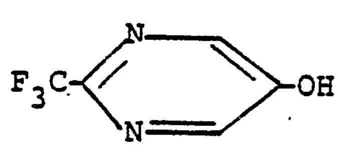

(II)

ou les sels correspondants de métaux alcalins, de métaux alcalino-terreux ou d'ammonium, avec des halogénures de formule (III)

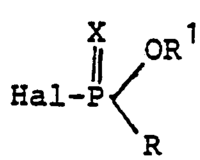

(III)

dans laquelle

R, $R^1$ et X ont les significations indiquées ci-dessus, et
Hal représente un atome d'halogène (de préférence un atome de chlore ou un atome de brome)

éventuellement en présence d'un accepteur d'acide et éventuellement en présence d'un diluant.

6. Agent parasiticide, caractérisé en ce qu'il contient au moins un composé de formule (I) selon la revendication 1 ou 5.

7. Utilisation de composés de formule (I) selon la revendication 1 ou 5, en vue de combattre les parasites, en particulier, les insectes et les nématodes.

8. Procédé en vue de combattre les parasites caractérisé en ce qu'on fait agir des composés de formule (I), selon la revendication 1 ou 5, sur les parasites, de préférence, les insectes et les nématodes, ou sur leurs biotopes.

9. Procédé de préparation d'agents parasiticides, caractérisé en ce qu'on mélange des composés de formule (I), selon la revendication 1 ou 5, avec des diluants et/ou des agents tensioactifs.